(19) Europäisches Patentamt
European Patent Office
Office européen des brevets

(11) Publication number : **0 455 789 B1**

(12) **EUROPEAN PATENT SPECIFICATION**

(45) Date of publication of patent specification : **16.03.94 Bulletin 94/11**

(51) Int. Cl.$^5$ : **A61K 31/495**

(21) Application number : **90917677.8**

(22) Date of filing : **19.11.90**

(86) International application number : **PCT/EP90/01985**

(87) International publication number : **WO 91/07967 13.06.91 Gazette 91/13**

# (54) METHOD OF PREVENTING OR LIMITING REPERFUSION DAMAGE.

Divisional application 93201656.1 filed on 19/11/90.

(30) Priority : **22.11.89 US 440142**

(43) Date of publication of application : **13.11.91 Bulletin 91/46**

(45) Publication of the grant of the patent : **16.03.94 Bulletin 94/11**

(84) Designated Contracting States : **AT BE CH DE DK ES FR GB GR IT LI LU NL SE**

(56) References cited :
**EP-A- 0 068 544**
**J. Mol. Cell. Cardiol., vol. 21, suppl. 2, 1989, (London, GB), H. van Belle et al., p. 142, abstract 424**
**Br. J. Pharmacol., vol. 99, Proc. Suppl., 1990, (GB), C.L. Wainwright et al., abstract 14P**

(56) References cited :
**Eur. J. of Pharmacology - Molecular Pharmacology Section, vol 172, 1989, Elsevier Science Publishers B.V. (Biomedical Division), (Amsterdam, NL), A.P. Ijzerman et al., pp. 273-281**
**J. Mol. Cell. Cardiol., vol. 21, no. 8, Aug. 1989, pp. 797-805**
**Med. Sci. Res., vol. 17, no. 20, 16-31 Oct. 1989, pp. 845-846**

(73) Proprietor : **JANSSEN PHARMACEUTICA N.V.**
**Turnhoutseweg 30**
**B-2340 Beerse (BE)**

(72) Inventor : **VAN BELLE, Herman**
**Vossenlaan 3**
**B-2340 Beerse (BE)**
Inventor : **VAN LAERHOVEN, Willy, J., C.**
**Sint-Corneliusstraat 58**
**B-2340 Beerse (BE)**

(74) Representative : **Wante, Dirk**
**Janssen Pharmaceutica N.V., Patent Department, Turnhoutseweg 30**
**B-2340 Beerse (BE)**

**EP 0 455 789 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

A recurring problem during reperfusion, i.e. the restoration of blood flow through tissue or organs previously deprived of blood supply, (e.g. after thrombolysis, in hearts after open heart surgery or in hearts for transplantation) is the further degeneration of this tissue or organ by leukocytes and their cytotoxic products.

The present invention provides a novel method of preventing or limiting reperfusion damage upon reperfusion of a transplanted heart by application of particular N-aryl-piperazinealkanamide derivatives and also a novel method for preserving hearts for transplantation significantly longer.

Some compounds which can be used in the present invention are known from U.S. Pat. No. 4,766,125 as agents useful for protecting the heart from myocardial injury caused by ischaemia, anoxia or hypoxia.

Some compounds are also described in U.S. Pat. No. 4,880,808 as useful therapeutical agents which improve sleep and counteract sleep disorders.

The present invention is concerned with the use of a N-aryl-piperazinealkanamide compound for the manufacture of a medicament for preventing or limiting reperfusion damage upon reperfusion of a transplanted heart, said compound having the formula

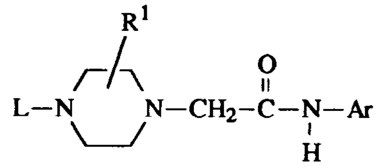 (I),

a stereochemically isomeric form thereof or a pharmaceutically acceptable acid addition salt form thereof, wherein

L is a radical of formula

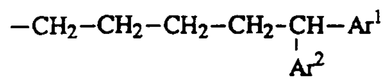 (a),

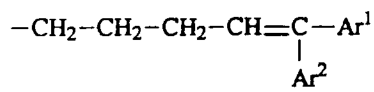 (b),

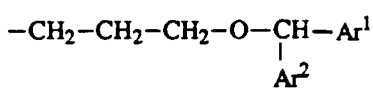 (c),

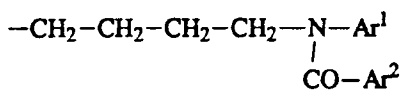 (d),

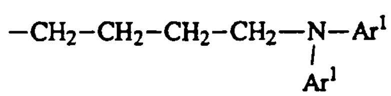 (e),

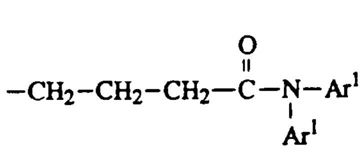 (f),

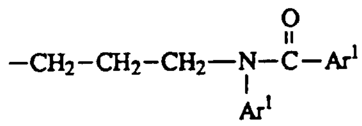 (g),

or

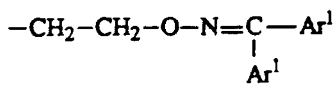 (h),

$A^{r1}$ is phenyl optionally substituted with halo or $C_{1-4}$alkyloxy;

$A^{r2}$ is phenyl optionally substituted with halo or $C_{1-4}$alkyloxy, or pyridinyl;

$R^1$ is $C_{1-4}$alkyl, aminocarbonyl or ($C_{1-4}$alkyl)aminocarbonyl;

Ar is a radical of formula

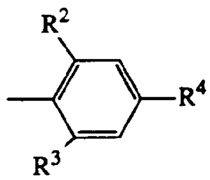 (i), 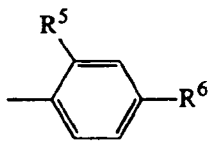 (j), 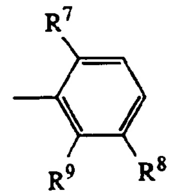 (k),

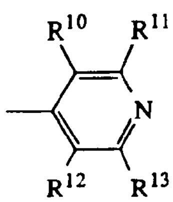 (l) or 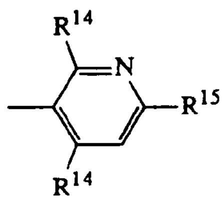 (m)

$R^2$ and $R^3$ each independently are halo or $C_{1-4}$alkyl;

$R^4$ is hydrogen, halo, nitro, amino, mono- or di($C_{1-4}$alkyl)amino, $C_{1-4}$alkylcarbonylamino, aminocarbonylamino, $C_{1-4}$alkyl, $C_{1-4}$alkyloxy, $C_{1-4}$alkyloxycarbonyl, $C_{1-4}$alkylcarbonyl, aminocarbonyl, mono- or di($C_{1-4}$alkyl)aminocarbonyl, cyano or aminomethyl;

$R^5$ is $C_{1-4}$alkylcarbonyl;

$R^6$ is hydrogen, amino, mono- or di($C_{1-4}$alkyl)amino, $C_{1-4}$alkylcarbonylamino, aminocarbonylamino, aminocarbonyl or cyano;

$R^7$ is $C_{1-4}$alkyl;

$R^8$ is halo or $C_{1-4}$alkylcarbonyl;

$R^9$ is hydrogen or $C_{1-4}$alkyl;

$R^{10}$ is halo or $C_{1-4}$alkyl;

$R^{11}$ is hydrogen, hydroxy or $C_{1-4}$alkyl;

$R^{12}$ is halo or $C_{1-4}$alkyl;

$R^{13}$ is hydrogen or

$R^{12}$ and $R^{13}$ taken together may also form a $C_{3-5}$alkanediyl radical;

each $R^{14}$ is $C_{1-4}$alkyl; and

$R^{15}$ is $C_{1-4}$alkyl or amino.

In the foregoing definitions the term halo is generic to fluoro, chloro, bromo and iodo; the term $C_{1-4}$alkyl defines straight and branched chain saturated hydrocarbon radicals having from 1 to 4 carbon atoms, such as,

for example, methyl, ethyl, propyl, 1-methylethyl, butyl or 1,1-dimethylethyl; the term $C_{3-5}$alkanediyl defines straight and branched chain saturated bivalent hydrocarbon radicals having from 3 to 5 carbon atoms, such as, for example, 1,3-propanediyl, 1,4-butanediyl and 1,5-pentanediyl.

The compounds of formula (I) wherein $R^{11}$ is hydroxy may also exist in the tautomeric oxo-form.

The compounds have at least one asymmetric carbon atom in their structure, namely the piperazine carbon atom bearing the $R^1$-radical which may be present in a R- or a S-configuration. Consequently, the compounds of formula (I) may be present in two different enantiomeric forms, which may be separated from each other, for example, by converting the mixture of enantiomers into the acid addition salt form thereof with an optically active acid, separating the diastereomeric salts, e.g., by selective crystallization, and liberating the pure enantiomers by treatment with alkali.

When L has one or more additional chiral centers, each of these chiral centers may be present in the R- or S-configuration and the compounds of formula (I) may have different diastereochemical forms, which may be separated from each other by physical separation methods such as, selective crystallization and chromatographic techniques, e.g. counter current distribution, columnchromatography and the like techniques.

Pure stereochemically isomeric forms may also be derived from the corresponding pure stereochemically isomeric forms of the appropriate starting materials, provided that the reaction occurs stereospecifically.

The compounds of formula (I) can be used as such or in a pharmaceutically acceptable acid addition salt form, the latter being conveniently obtained by treating the base form with an appropriate acid. Appropriate acids comprise, for example, inorganic acids such as hydrohalic acids, e.g. hydrochloric or hydrobromic acid; sulfuric acid; nitric acid; phosphoric acid and the like; or organic acids, such as, for example, acetic, propanoic, hydroxyacetic, 2-hydroxypropanoic, 2-oxopropanoic, ethanedioic, propanedioic, butanedioic, (Z)-2-butenedioic, (E)-2-butenedioic, 2-hydroxybutanedioic, 2,3-dihydroxybutanedioic, 2-hydroxy-1,2,3propanetricarboxylic, methanesulfonic, ethanesulfonic, benzenesulfonic, 4-methylbenzenesulfonic, cyclohexanesulfamic, 2-hydroxybenzoic, 4-amino-2-hydroxybenzoic and the like acids.

The term acid addition salt form as used hereinabove also comprises the solvates which the compounds of formula (I) and their acid addition salts are able to form. Examples of such solvates are e.g. the hydrates, alcoholates and the like.

Particular compounds for use in the present invention are those compounds wherein

$Ar^1$ is phenyl optionally substituted with fluoro or methoxy; and/or

$Ar^2$ is phenyl optionally substituted with fluoro or methoxy, or 3-pyridinyl; and/or

$R^1$ is methyl, aminocarbonyl or methylaminocarbonyl; and/or Ar is a radical of formula (i), (j), (l) or (m).

More particular compounds are those particular compounds wherein

$Ar^1$ is phenyl, 4-fluorophenyl or 4-methoxyphenyl; and/or

$Ar^2$ is phenyl, 4-fluorophenyl, 4-methoxyphenyl or 3-pyridinyl; and/or Ar is a radical of formula (i) or (j).

Particularly preferred compounds are those more particular compounds wherein L is 5,5-bis(4-fluorophenyl)pentyl, 5,5-bis(4-fluorophenyl)-4-pentenyl, 5-(4-fluorophenyl)-5-(3-pyridinyl)-4-pentenyl 4[N-(4-fluorophenyl)-N-(3-pyridinylcarbonyl)amino]butyl, N,N-bis(4-fluorophenyl)butanamide, 2-[[[bis(4-fluorophenyl)methylen]amino]oxy]ethyl or 3-[(4-fluorophenyl)(3-pyridinyl)methoxy]propyl; and/or $R^1$ is 2-methyl, 3-methyl, 2-aminocarbonyl, 3-aminocarbonyl or 3-methylaminocarbonyl; and/or

$R^2$ and $R^3$ are both chloro or methyl; and/or

$R^4$ is hydrogen, chloro, nitro, amino, dimethylamino, ethylcarbonylamino, aminocarbonylamino, methoxy, ethoxycarbonyl, acetyl, aminocarbonyl, dimethylaminocarbonyl, cyano or aminomethyl; or $R^5$ is acetyl; and/or $R^6$ is amino, dimethylamino, ethylcarbonylamino, aminocarbonylamino, aminocarbonyl or cyano.

Of particular interest are those enantiomeric forms of the compounds of the above defined groups, which selectively bind and block the nucleoside transport protein of cell membranes.

The most interesting compounds for use according to the present invention are (*l*)-(-)-2-(aminocarbonyl)-N-(4-amino-2,6-dichlorophenyl-)4-[5,5-bis(4-fluorophenyl)pentyl]-1-piperazineacetamide (I-b), the pharmaceutically acceptable acid addition salt forms thereof and the hydrated forms thereof, in particular the mono- and the hemihydrate thereof. Said most interesting compounds are novel and possess a unique combination of pharmacological selectivity and advantageous characteristics, which is not shared with the previously known N-aryl piperazinealkanamide compounds. Besides being potent, selective nucleoside transport inhibitors, they do not have $Ca^{2+}$-antagonistic properties. Their acute and chronic toxicity is very low. Furthermore, said compounds do not bind to any large extent to plasma proteins and consequently their bioavailability is excellent. Greatly simplifying and broadening their application in therapy, is the fact that they are easily resorbed and thus can be administered orally. In conjunction with the above properties, this convenient route of administering said compounds, renders them particularly suitable for prolonged use, e.g. as prophylactics in high risk patients or in maintenance therapy as set forth hereinafter. A further advantage of using enantiomerically pure

compounds resides in the fact that the amount of drug to be administered may be lowered because the undesired inactive enantiomorph is not any longer present. This approach is particularly advantageous as it reduces the likelihood of overdosing and its potentially undesired side-effects such as cardiodepression at said overdoses.

Further most interesting compounds for use according to the present invention are the compounds of formula (I-c), as depicted herebelow, wherein the configuration at the carbon atom bearing the radical $R^1$ is the same as in the abovementioned compound (I-b). The compounds of formula (I-c) are deemed novel too.

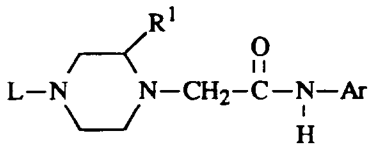 (I-c).

The compounds of formula (I), the stereochemically isomeric forms and the pharmaceutically acceptable acid addition salts thereof, wherein L, $R^1$ and Ar are as defined hereinabove, and wherein

1) L is a radical of formula (h), or

2) Ar is a radical of formula (m) wherein $R^{15}$ is amino, said compounds being represented by formula (I-a) and said radicals L and Ar being represented respectively by $L^a$ and $Ar^a$

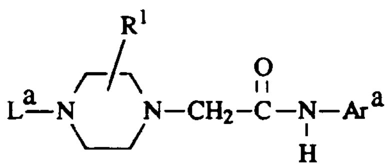 (I-a),

are also novel.

Interesting novel compounds are 3-(aminocarbonyl)-4-[5,5-bis(4-fluorophenyl)- pentyl]-N-(6-amino-2,4-dimethyl-3-pyridinyl)-1-piperazineacetamide hemihydrate and 2-(aminocarbonyl)-4-[2-[[[bis(4-fluorophenyl)methylene]amino]oxy]ethyl]-N-(2,6-dichlorophenyl)-1-piperazineacetamide.

A number of compounds of formula (I) as well as their syntheses and their pharmacological properties, are known from U.S. Patent No. 4,766,125 and EP-A-0,285,219. The novel enantiomerically pure compounds of formula (I) can conveniently be prepared from enantiomerically pure piperazines of formula

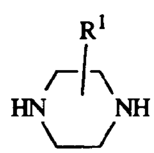 (II)

wherein $R^1$ is as defined under formula (I), and wherein one or two piperazine nitrogen atoms may optionally be protected with a selectively removable group such as, for example, a 1-aryl alkyl group, e.g., phenylmethyl, 1-phenylethyl and the like, an (aryl or $C_{1-4}$alkyl)oxycarbonyl group, e.g. phenoxycarbonyl, methoxycarbonyl, ethoxycarbonyl, tert. butyloxycarbonyl and the like protecting groups. Said protective groups can be removed following art-known procedures, such as hydrogenolysis and hydrolysis.

Said preparation generally involves the consecutive N-alkylation or reductive N-alkylation of each piperazine nitrogen atom with the appropriate L-moiety or N-arylalkanamide moiety following art-known procedures. Typically said preparation proceeds as follows:

(a) in unprotected piperazine derivatives, the most reactive, least sterically hindered nitrogen at the 4-position is N-alkylated first and then the remaining free nitrogen atom at the original 1-position of the starting piperazine is reacted;

(b) in monoprotected piperazine derivatives, the free nitrogen atom is N-alkylated, the protective group is removed and the now unprotected nitrogen atom is reacted; or

(c) in bis-protected piperazine derivatives, both protective groups are removed and one proceeds as under (a), or one group is selectively removed, whereupon one proceeds as in (b).

For example, the novel compound (*l*)-(-)-2-(aminocarbonyl)-N-(4-amino-2,6-dichlorophenyl)-4-[5,5-bis(4-fluorophenyl)pentyl]-1-piperazineacetamide of formula

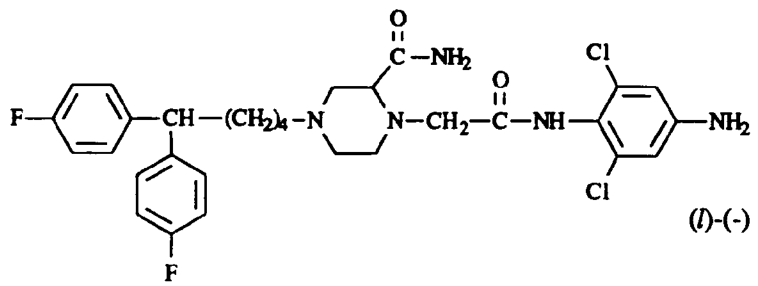

(*l*)-(-) (I-b)

can conveniently be prepared starting from (-)-(S,S)-N[1],N[2]-bis(1-phenylethyl)-1,2-ethanediamine (II).

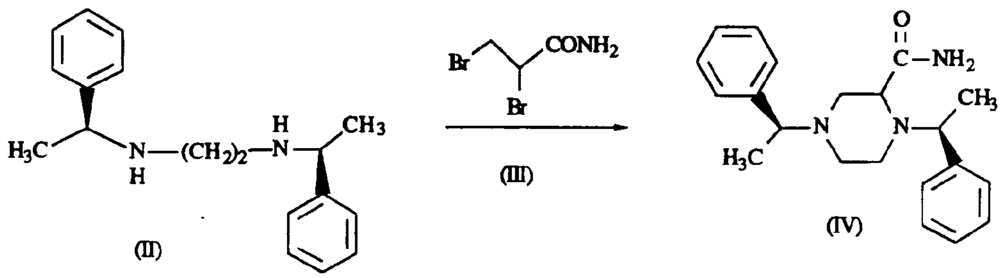


(II) (IV)

The intermediate (II) is cyclized to a piperazine derivative by double N-alkylation with 2,3-dibromopropanamide (III) in a reaction-inert solvent in the presence of a base. Appropriate solvents are aromatic hydrocarbons, e.g. benzene, methylbenzene and the like, halogenated hydrocarbons e.g. tetrachloromethane, chlorobenzene and the like. Suitable bases are alkali and earth alkaline metal carbonates such as, for example, sodium and potassium carbonate. Said cyclization is preferably carried out at the reflux temperature of the reaction mixture.

The intermediate of formula (IV) is converted into the piperazine (+)-(V) by hydrogenolysis under a hydrogen atmosphere in an alkanol such as, for example, methanol, ethanol and the like, and in the presence of a hydrogenation catalyst such as palladium-on-charcoal, platinium-on-charcoal and the like.

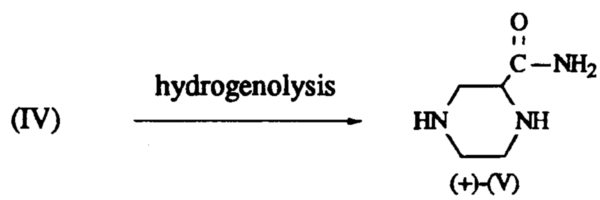


(IV) (+)-(V)

Next the intermediate (+)-(V) is reductively N-alkylated by reaction with 5,5-bis(4-fluorophenyl)pentaldehyde (VI) under a hydrogen atmosphere in an alkanol such as, for example, methanol, ethanol and the like, in the presence of a hydrogenation catalyst such as, for example, palladium-on-charcoal, platinum-on-charcoal and the like. In order to prevent the further reaction of the reaction product it is advantageous to add a catalyst poison such as thiophene to the reaction mixture. In order to enhance the rate of the reaction, the reaction mixture is heated slightly, in particular to about 40°C to 60°C.

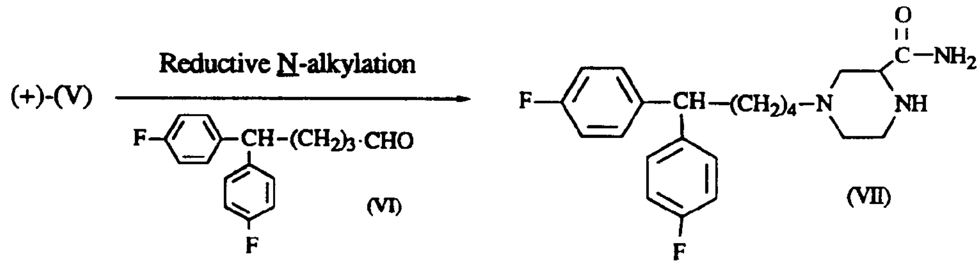


(+)-(V) (VI) (VII)

Alternatively, intermediate (VII) can be prepared by N-alkylating intermediate (+)-(V) with a 5,5-bis(4-fluorophenyl)pentane-1-halide or sulfonate of formula

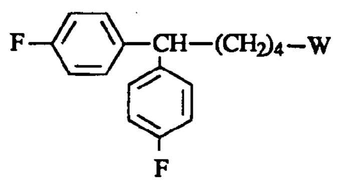 (VI-a)

wherein W is halo, e.g. chloro or bromo, or sulfonyloxy, e.g. methanesulfonyloxy, 4-methylbenzenesulfonyloxy and the like. Said alkylation can conveniently be conducted in a reaction-inert solvent such as, for example, an alkanol, e.g. methanol, butanol, cyclohexanol and the like, a dipolar aprotic solvent, e.g. N, N-dimethylformamide, dimethylsulfoxide and the like, in the presence of a base, e.g. an alkali or earth alkaline metal hydroxide, carbonate, e.g. sodium or potassium hydroxide or carbonate.

The thus obtained intermediate (VII) is N-alkylated with a reagent of formula (VIII) wherein W represents a reactive leaving group such as chloro, bromo and the like, in a reaction-inert solvent in the presence of a base.

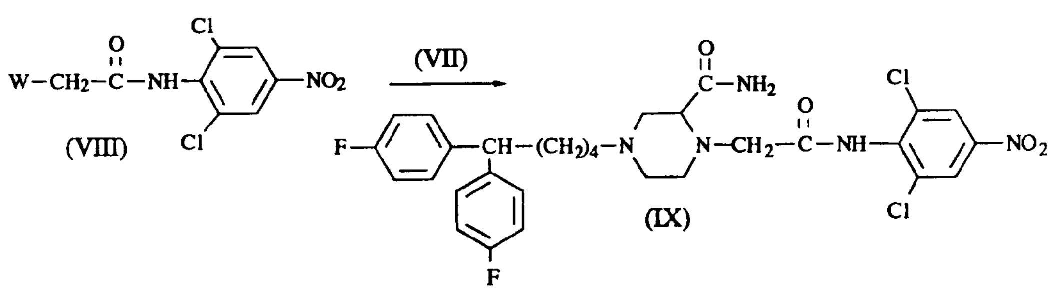

(VIII) (VII) (IX)

Said N-alkylation reaction can conveniently be conducted by stirring and heating the reactants, in particular by heating at about 70°C to 100°C. Suitable solvents are, for example, alkanols, e.g., methanol, ethanol, butanol and the like, dipolar aprotic solvents, e.g. N,N-dimethylformamide, dimethyl sulfoxide and the like, or mixtures of said solvents. Appropriate bases are alkali and earth alkaline metal hydroxides, carbonates, hydrogen carbonates and the like, and organic amines, such as, for example, N,N-diethylethanamide, pyridine, morpholine and the like bases. An alkali metal iodide, e.g. potassium iodide may be added in order to enhance the reaction rate.

The intermediate of formula (IX) is finally converted to the novel compound (*l*)-(-)(I-b) by a nitro-to-amino reduction step.

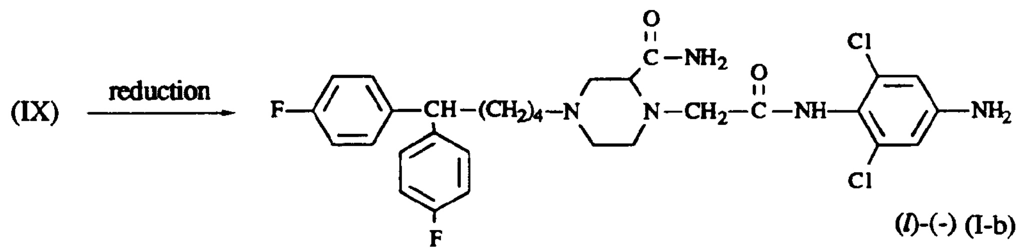


(*l*)-(-) (I-b)

Said reduction can conveniently be conducted in a reaction-inert solvent following art-known reduction procedures. For example, intermediate (IX) may be stirred under a hydrogen atmosphere in an alkanol such as, methanol, ethanol and the like, in the presence of a hydrogenation catalyst such as palladium-oncharcoal, platinum-on-charcoal, Raney nickel and the like. Alternatively said reduction may also be accom-plished by treatment of the intermediate (IX) within a reagent such as, for example, sodium sulfite, sodium sulfide, sodium hydrogen sulfide, titanium (III) chloride and the like.

The other novel compounds of formulae (I-a) and (I-c) as defined hereinabove are prepared following the procedure described hereinbefore. Thus, a piperazine of formula (V-a).

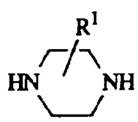

is N-alkylated with an appropriate alkylating reagent L-W (VI-b) or is reductively N-alkylated with a reagent $L^1$=O (VI-c), said $L^1$=O representing a reagent of formula L-H wherein two geminal hydrogen atoms are replaced by an oxo-group, following the reaction procedures described in detail hereinabove. The thus obtained intermediate

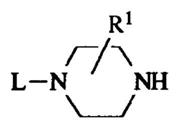

(VII-a)

is then N-alkylated with an appropriate N-aryl alkanamide of formula

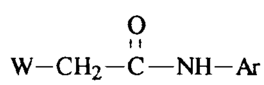

(VIII-a) following the N-alkylation procedure described hereinabove.

In all of the preceding reaction steps, the intermediates and final compounds may be isolated and purified following art-known procedures, in particular by liquid chromatography and crystallization.

As previously mentioned the compounds of formula (I) are known to protect the heart from myocardial injury and to improve sleep and counteract sleep disorders. A number of said compounds, more particularly 3-(aminocarbonyl)-4-[4,4-bis(4-fluorophenyl)-butyl]-N-(2,6-dichlorophenyl)-1-piperazineacetamide dihydrochloride, monohydrate, generically known as mioflazine, is described as an inhibitor of nucleoside transport through membranes [Molecular Physiology, 8, 615-630 (1985)]. An important, common advantage of the present compounds of formula (I) for use in the methods described hereinbelow is the fact that they are orally active and, contrary to mioflazine, have an excellent bioavailability because they do not generally bind to plasma proteins.

The term reperfusion damage, also designated reperfusion injury, as used in the instant application defines the damage to tissues and organs which have been previously deprived of blood supply upon reperfusion, i.e. the restoration of blood flow through said tissues and organs. Reperfusion damage is an acute phenomenon which arises immediately upon reperfusion and therefore must be attended to timely.

Compositions for preventing or limiting reperfusion damage upon reperfusion of a transplanted heart comprise a pharmaceutically acceptable carrier and as active ingredient an effective reperfusion damage preventing or limiting amount of a compound of formula (I).

Interesting compositions among the compositions described hereinbefore and -after, are those comprising a cyclodextrin (CD) or an ether derivative thereof, as a complexant and/or solubilizer. As examples of such cyclodextrins there may be mentioned α-CD, β-CD, γ-CD, and ether or mixed ether derivatives thereof. Particular such cyclodextrin derivatives are described in US-3,459,731, EP-A-0,149,197 and EP-A-0,197,571.

Typically such ether or mixed ether derivatives comprise α-,β or γ-CD wherein one or more hydroxylgroups are substituted with $C_{1-6}$alkyl, particularly methyl, ethyl or isopropyl; hydroxy$C_{1-6}$alkyl, particularly hydroxyethyl, hydroxypropyl or hydroxy-butyl; carboxy$C_{1-6}$alkyl, particularly carboxymethyl or carboxyethyl; or $C_{1-6}$alkyloxycarbonyl$C_{1-6}$alkyl. Especially noteworthy as complexants and/or solubilizers are β-CD, 2,6-dimethyl-β-CD and in particular 2-hydroxypropyl-β-CD, 2-hydroxyethyl-β-CD, 2-hydroxyethyl-γ-CD and 2-hydroxypropyl-γ-CD. In the aforementioned cyclodextrin derivatives, the DS (degree of substitution, i.e. the average number of substituted hydroxy functions per glucose unit) preferably is in the range of 0.125 to 3, in particular 0.3 to 2, more in particular 0.3 to 1. The MS (molar degree of substitution, i.e. the average number of moles of the substituting agent per glucose unit) is in the range of 0.125 to 10, in particular 0.3 to 3 and more in particular 0.3 to 1.5, preferably 0.35 to 0.50. Said compositions may conveniently be prepared by dissolving the cyclodextrin or ether derivative thereof in water and adding thereto a compound of formula (I) as well as other adjuvants and components such as, for example, sodium chloride, potassium nitrate, glucose, mannitol, sorbitol, xylitol and buffers such as, for example, phosphate, acetate or citrate buffers; and optionally concentrating or drying the solution by evaporation under reduced pressure or by lyophilization. The amount of the cyclodextrin or ether derivative thereof in the final composition generally ranges from about 1% to about 40% by weight, particularly from 2.5% to 25% and more particularly from 5 % to 20%. The amount of the active ingredient of formula (I) in said final compositions generally ranges from about 0.01% to about 1.0% by weight, particularly from 0.025% to 0.5% and more particularly from 0.05% to 0.2%. Particularly interesting compositions are those comprising (*l*)-(-)2-(aminocarbonyl)-N-(4-amino-2,6-dichlorophenyl)-4-[5,5-bis(4-fluorophenyl)pentyl]-1-piperazineacetamide as active ingredient and 2-hydroxypropyl-β-cyclodextrin as complexant and/or solubilizer.

To prepare the pharmaceutical compositions of this invention, an effective amount of the active ingredients, in acid or base addition salt form or base form, is combined in intimate admixture with a pharmaceutically acceptable carrier, which can take a wide variety of forms depending on the form of preparation desired for administration. These pharmaceutical compositions are desirably in unitary dosage form suitable, preferably, for administration orally, rectally, percutaneously, or by parenteral injection. For example, in preparing the compositions in oral dosage form, any of the usual pharmaceutical media may be employed, such as, for example, water, glycols, oils, alcohols and the like in the case of oral liquid preparations such as suspensions, syrups, elixirs and solutions; or solid carriers such as starches, sugars, kaolin, lubricants, binders, disintegrating agents and the like in the case of powders, pills, capsules and tablets. Because of their ease in administration, tablets and capsules represent the most advantageous oral dosage unit form, in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. In the compositions suitable for percutaneous administration, the carrier optionally comprises a penetration enhancing agent and/or a suitable wettable agent, optionally combined with suitable additives of any nature in minor proportions, which additives do not cause any significant deleterious effects on the skin. Said additives may facilitate the administration to the skin and/or may be helpful for preparing the desired compositions. These compositions may be administered in various ways, e.g., as a transdermal patch, as a spot-on or as an ointment. Acid addition salts of (I) due to their increased water solubility over the corresponding base form, are obviously more suitable in the preparation of aqueous compositions.

An especially interesting feature of the compounds of formula (I) is the fact that said compounds can be administered orally thus substantially simplifying the administration of said compounds to high risk patients.

It is especially advantageous to formulate the aforementioned pharmaceutical compositions in dosage unit form for ease of administration and uniformity of dosage. Dosage unit form as used in the specification and claims herein refers to physically discrete units suitable as unitary dosages, each unit containing a predetermined quantity of active ingredient calculated to produce the desired therapeutic effect, in association with the required pharmaceutical carrier. Examples of such dosage unit forms are tablets (including scored or coated tablets), capsules, pills, powder packets, wafers, injectable solutions or suspensions, teaspoonfuls, tablespoonfuls and the like, and segregated multiples thereof.

The term transplantation as used herein defines the transplantation of tissues, in particular of organs and more in particular of hearts from one warm-blooded animal to an identical recipient site within another warm-blooded animal, said warm-blooded animals in particular being humans. The present invention particularly provides a method of preventing or limiting reperfusion damage upon reperfusion of a transplanted heart, said method comprising administering to the patient receiving the donor heart, as well as administering to the heart to be transplanted an effective amount of a compound of formula (I).

More in particular, said method comprises arresting a donor heart for transplantation with a cardioplegic solution, whereby said cardioplegic solution comprises an effective amount of a compound of formula (I), storing said heart in the cold in said cardioplegic solution and transplanting said heart in another subject pre-treated with an effective amount of compound of formula (I) and subsequently reperfusing said heart with oxygenated blood.

The term cardioplegic solution as used herein defined the normal balanced-salt formulations generally used for cardioplegia. Commonly used cardioplegic solutions for arresting a heart comprise for example, hyperkalemic NIH cardioplegic solutions, UW cardioplegic solution, Collins cardioplegic solution M (115 meq $K^+$/L), St. Thomas' Hospital cardioplegic solution, Ringer's injection buffered with tromethamine (3.6%), Plegisol® (Abbott) buffered with sodium hydrogen carbonate injection (8.4%), Modified Krebs high K (34 meq/L) solution and the like cardioplegic solutions. Typical temperatures for storing a heart for transplantation may range from 0°C to about 10°C, particularly from about 0°C to about 7°C and more particularly from about 0°C to about 4°C.

The amount of each of the active ingredients, the cardioplegic solution and the compound of formula (I), in the method according to the present invention is such that effective cardioplegia is obtained upon administration, concomitant with effective prevention or limitation of reperfusion damage upon reperfusion of the transplanted heart. For example, the amount of the compound of formula (I) in the cardioplegic solution for use in the present method may typically range from about 0.1 μM to about 10 μM, particularly from about 0.5 μM to about 5 μM and more particularly from about 0.8 μM to about 2 μM.

The amount of the active ingredient, the compound of formula (I), upon administration to the patient receiving a donor heart is such that effective prevention or limitation of reperfusion damage upon reperfusion of the transplanted heart is obtained. For example, the amount of the compound of formula (I) for use in the present method may typically range from about 0.01 to about 100 mg/kg bodyweight, particularly from about 0.1 to about 10 mg/kg bodyweight and more particularly from about 0.2 to about 5 mg/kg bodyweight.

In still a further aspect of the present invention there is provided a method of storing a heart for transplantation in a cardioplegic solution in the cold, which method comprises administering to said cardioplegic solution an amount, effective in prolonging the storage of said heart, of a compound of formula (I) as defined hereinabove.

An especially interesting feature of the present method of storing a heart for transplantation, is the fact that the duration of successfully storing a heart for transplantation in a cardioplegic solution comprising a compound of formula (I) can be prolonged drastically. Whereas a heart for transplantation can be stored successfully for about 4 hours in a usual cardioplegic solution without a compound of formula (I) in the cold, the novel method of storing a heart for transplantation in a cardioplegic solution comprising a compound of formula (I) in the cold allows one to store said heart for at least 24 hours and to transplant said heart subsequently successfully. Thus the present invention further provides a method of storing a heart for transplantation in the cold in a cardioplegic solution, particularly a cardioplegic solution specifically mentioned hereinabove, comprising an effective donor heart protecting amount of a compound of formula (I). The effective donor heart protecting amount of the compound of formula (I) in the cardioplegic solution for use in the present method of storing a heart for transplantation may typically range from about 0.1μM to about 10μM, particularly from about 0.5μM to about 5μM and more particularly from about 0.8μM to about 2μM.

## EXPERIMENTAL PART

Example 1 Preparation of (-)-2-(aminocarbonyl)-N-(4-amino-2,6-dichlorophenyl)-4-[5,5-bis(4-fluorophenyl)-pentyl]-1-piperazineacetamide

A mixture of 108.4 parts of (-)-(S,S)-$N^1$,$N^2$-bis(1-phenylethyl)-1,2-ethanediamine, 93.8 parts of 2,3-dibromopropanamide, 334.5 parts of potassium carbonate and 2958 parts of methylbenzene was refluxed for 24 hours using a water separator. The reaction mixture was filtered while hot and the precipitate was partitioned between water and dichloromethane. The organic layer was separated and combined with the filtrate. The whole was dried, filtered and evaporated. The residue was purified by column chromatography (Lichroprep RP18; $H_2O$ (0.5% $CH_3COONH_4$ ) / $CH_3CN$ 55:45). The eluent of the desired fraction was evaporated, yielding 20.8 parts (15.4%) of [1(S), 2A, 4(S)]-1,4-bis(1-phenylethyl)-2-piperazinecarboxamide (interm. 1).

A mixture of 20.8 parts of intermediate (1) and 198 parts of methanol was hydrogenated at normal pressure and room temperature with 2 parts of palladium-on-charcoal catalyst 10%. After the calculated amount of hydrogen was taken up, the catalyst was filtered off and the filtrate was evaporated, yielding 7.8 parts (98.0%) of (+)-2-piperazine-carboxamide (interm. 2).

A mixture of 3.9 parts of intermediate (2), 8.3 parts of 5,5-bis(4-fluorophenyl)-pentaldehyde, 2 parts of a solution of thiophene in methanol (4%) and 198 parts of methanol was hydrogenated at normal pressure and 50°C with 2 parts of palladium-on-charcoal catalyst 10%. After the calculated amount of hydrogen was taken up, the catalyst was filtered off and the filtrate was evaporated. The residue was converted into the ethanedioate salt in ethanol. The salt was recrystallized from a mixture of ethanol and methanol. The product was filtered off and dried, yielding 8.82 parts (61.6%) of (+)-4-[5,5-bis(4-fluorophenyl)pentyl]-2-piperazinecarboxamide ethanedioate (1:1); $[\alpha]_D^{20}$ = +10.02° (conc. = 0.5% in DMF) (interm. 3).

8.82 Parts of intermediate (3) were taken up in water and converted into the free base with $NH_4OH$. The base was extracted with dichloromethane (3x) and the combined extracts were dried, filtered and evaporated. To the residue there were added 6.5 parts of 1-chloro-N-(2,6-dichloro-4-nitrophenyl)acetamide, 3.75 parts of N,N-diethylethanamine and 113 parts of N,N-dimethylformamide. The whole was stirred over weekend at 70°C and was then evaporated. The residue was partitioned between $NaHCO_3$ (5% aq.) and dichloromethane. The organic layer was separated, dried, filtered and evaporated. The residue was purified by column chromatography (silica gel; $CH_2Cl_2$ / ($CH_2Cl_2$ + 10% $CH_3OH$) 70:30). The eluent of the desired fraction was evaporated and the residue was converted into the hydrochloride salt in 2-propanol. The product was filtered off and dried in vacuo at 50°C, yielding 3.78 parts (30.5%) of (-)-2-(aminocarbonyl)-4-[5,5-bis(4-fluorophenyl)pentyl]-N-(2,6-dichloro-4-nitrophenyl)-1-piperazineacetamide monohydrochloride; $[\alpha]_D^{20}$ = -18.47° (conc. = 0.5% in $CH_3OH$) (interm. 4).

A mixture of 3.6 parts intermediate (4), 1 part of a solution of thiophene in methanol (4%) and 119 parts of methanol was hydrogenated at normal pressure and room temperature with 2 parts of palladium-on-charcoal catalyst 10%. After the calculated amount of hydrogen was taken up, the catalyst was filtered off and the filtrate was evaporated. The residue was purified by column chromatography (Lichroprep RP-18; $H_2O$ (0.5% $CH_3COONH_4$) / $CH_3OH$ / $CH_3CN$ 40:20:40). The desired fractions were concentrated and the product was allowed to crystallize from the resulting aqueous solution. It was filtered off and dried in vacuo at 60°C, yielding 1.49 parts (43.4%) of (-)-2-(aminocarbonyl)-N-(4-amino-2,6-dichlorophenyl)-4-[5,5-bis(4-fluorophenyl)pentyl]-1-piperazineacetamide hemihydrate; mp. 123.4°C; $[\alpha]_D^{20}$ = -29.63° (conc. =0.5% in $CH_3OH$) (comp. I-b).

Example 2 : Biological Example

Dog hearts were arrested either with hyperkalemic NIH cardioplegia (group I, n=6) or with the same cardioplegia after addition of 2-(aminocarbonyl)-N-(4-amino-2,6-dichlorophenyl)-4-[5,5-bis(4-fluorophenyl)pentyl]-1-piperazineacetamide (group II, n=6). The hearts were stored cold for 24 hours at 0-5°C (ice-water) in the cardioplegic solution and then transplanted orthotopically. To the recipient dogs was administered 0.1 mg/kg bodyweight of 2-(aminocarbonyl)-N-(4-amino-2,6-dichlorophenyl)-4-[5,5-bis(4-fluorophenyl)pentyl]-1-piperazineacetamide before transplantation.

Myocardial content of high energy phosphates (HEP) was determined in serial biopsies. In group I ATP was 50% and CrP 18% of control after 24 hours storage. During 60 minutes reperfusion on cardiopulmonary bypass (CPB) HEP content decreased ($p<0.05$) and all animals developed a "stone heart" after transplantation. After 24 hours storage in group II ATP was 82% and CrP 28% of control ($p<0.05$ vs. group I). After transplantation HEP content remained stable and all hearts could be weaned from CPB without inotropic support except for isoprenaline. Thus, optimal myocardial preservation was obtained with the combination of cardioplegia and nucleoside transport inhibition.

Example 3 : Composition Examples

## 1. Injectable Solution

| | |
|---|---|
| active ingredient * | 1g |
| hydrochloric acid 0.1N | 0.04 l |
| 2-hydroxypropyl-β-cyclodextrin | 50g |
| sodium chloride | 5.5g |
| sodium hydroxide 1N | ad pH 3.7 - 3.9 |
| water | ad 1 l |

* 2-(aminocarbonyl)-N-(4-amino-2,6-dichlorophenyl)-4-[5,5-bis(4-fluorophenyl)-pentyl]-1-piperazineacetamide.

### Method of preparation

50 g of HP-β-cyclodextrin are dissolved in 0.5 l of water. There are added successively 0.04 l of hydrochloric acid 0.1N and 1 g of 2-(aminocarbonyl)-N-(4-amino-2,6-dichlorophenyl)-4-[5,5-bis(4-fluorophenyl)pentyl]-1-piperazineacetamide. The whole is stirred until a clear solution is obtained. After diluting with water to 0.9 1, there are dissolved 5.5 parts of sodium chloride with stirring. The acidity is adjusted with sodium hydroxide 1N to pH 3.7 - 3.9. The solution is diluted with water to 1 l, thus yielding an injectable solution containing 1 mg/ml of active ingredient.

## 2. Oral Solution

| | |
|---|---|
| active ingredient * | 1g |
| 2-hydroxypropyl-β-cyclodextrin | 50g |
| hydrochloric acid 0.1N | 0.04 l |
| sorbitol 70% | 0.1 l |
| propyleneglycol | 0.1 l |
| disodium ethylenediaminetetraacetate | 2g |
| benzoic acid | 3g |
| mouth wash flavour | 1g |
| sodium hydroxide 1N | ad pH 4.0 |
| purified water | ad 1 l |

* 2-(aminocarbonyl)-N-(4-amino-2,6-dichlorophenyl)-4-[5,5-bis(4-fluorophenyl)-pentyl]-1-piperazineacetamide.

### Method of preparation

50 g of HP-β-cyclodextrin are dissolved in 0.6 l of water. There are added successively 0.04 l of hydrochloric

acid 0.1N and 1 g of 2-(aminocarbonyl)-N-(4-amino-2,6-dichlorophenyl)-4-[5,5-bis(4-fluorophenyl)pentyl]-1-piperazineacetamide. The whole is stirred until a clear solution is obtained. There are dissolved 2g of $Na_2$(EDTA) with stirring and then there is added 0.1 l of sorbitol 70%. To the homogeneous solution there are successively added a solution of 3g of benzoic acid in 0.1 l of propyleneglycol and 1g of mouth wash flavour. The acidity is adjusted with sodium hydroxide 1N to pH 3.7 - 3.9. The solution is diluted with water to 1 l, thus yielding an oral solution containing 1 mg/ml of active ingredient.

## Claims

1. The use of a N-aryl-piperazinealkanamide compound for the manufacture of a medicament for preventing or limiting reperfusion damage upon reperfusion of a transplanted heart, said compound having the formula

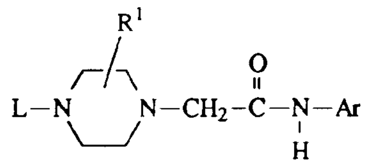 (I),

a stereochemically isomeric form or a pharmaceutically acceptable acid addition salt form thereof, wherein

L is a radical of formula

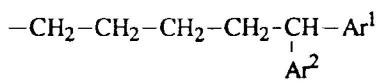 (a),

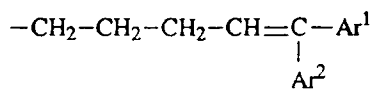 (b),

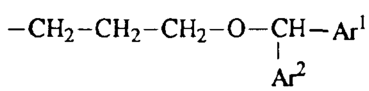 (c),

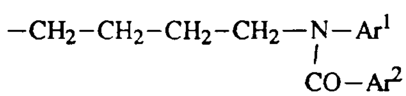 (d),

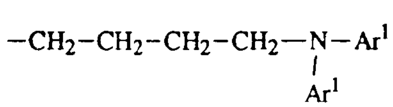 (e),

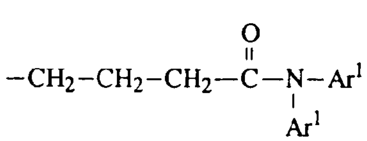 (f),

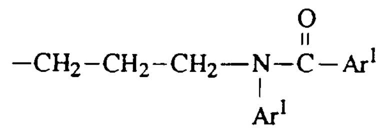 (g)

or

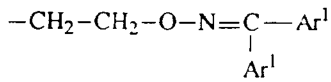 (h),

$Ar^1$ is phenyl optionally substituted with halo or $C_{1-4}$alkyloxy;
$Ar^2$ is phenyl optionally substituted with halo or $C_{1-4}$alkyloxy, or pyridinyl;
$R^1$ is $C_{1-4}$alkyl, aminocarbonyl or ($C_{1-4}$alkyl)aminocarbonyl;
Ar is a radical of formula

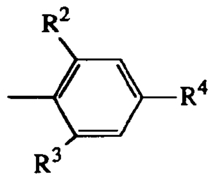 (i), 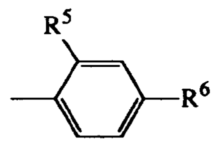 (j), 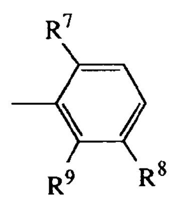 (k),

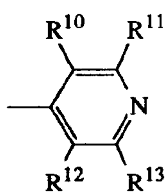 (l) or 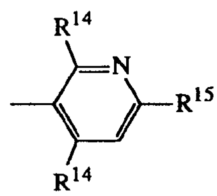 (m)

$R^2$ and $R^3$ each independently are halo or $C_{1-4}$alkyl;
$R^4$ is hydrogen, halo, nitro, amino, mono- or di($C_{1-4}$alkyl)amino, $C_{1-4}$alkylcarbonylamino, aminocarbonylamino, $C_{1-4}$alkyl, $C_{1-4}$alkyloxy, $C_{1-4}$alkyloxycarbonyl, $C_{1-4}$alkylcarbonyl, aminocarbonyl, mono- or di($C_{1-4}$alkyl)aminocarbonyl, cyano or aminomethyl;
$R^5$ is $C_{1-4}$alkylcarbonyl;
$R^6$ is hydrogen, amino, mono- or di($C_{1-4}$alkyl)amino, $C_{1-4}$alkylcarbonylamino, aminocarbonylamino, aminocarbonyl or cyano;
$R^7$ is $C_{1-4}$alkyl;
$R^8$ is halo or $C_{1-4}$alkylcarbonyl;
$R^9$ is hydrogen or $C_{1-4}$alkyl;
$R^{10}$ is halo or $C_{1-4}$alkyl;
$R^{11}$ is hydrogen, hydroxy or $C_{1-4}$alkyl;
$R^{12}$ is halo or $C_{1-4}$alkyl;
$R^{13}$ is hydrogen or
$R^{12}$ and $R^{13}$ taken together may also form a $C_{3-5}$alkanediyl radical;
each $R^{14}$ is $C_{1-4}$alkyl; and
$R^{15}$ is $C_{1-4}$alkyl or amino.

**2.** Use of a compound according to claim 1 for the manufacture of a medicament for protecting a donor heart for transplantation during storage.

**3.** Use according to claim 1 or claim 2 wherein the compound is (-)-2-(aminocarbonyl)- N-(4-amino-2,6-dichlorophenyl)4-[5,5- bis(4-fluorophenyl)pentyl]-1-piperazineacetamide monohydrate.

4. A method of protecting a donor heart for transplantation during storage, said method comprising arresting the heart for transplantation with a cardioplegic solution and storing said heart in the cold in a cardioplegic solution, whereby said cardioplegic solution comprises an effective donor heart protecting amount of a compound having the formula (I) as defined in claim 1.

5. A method according to claim 4 wherein the compound is (-)-2-(aminocarbonyl)-N-(4-amino-2,6-dichlorophenyl)-4-[5,5-bis(4-fluorophenyl)pentyl]-1-piperazineacetamide monohydrate.

## Patentansprüche

1. Verwendung einer N-Aryl-piperazinalkanamidverbindung zur Herstellung eines Medikamentes zur Verhütung oder Begrenzung einer Reperfusionsschädigung bei der Reperfusion eines transplantierten Herzens, welche Verbindung die Formel

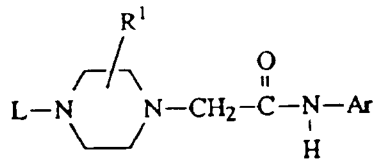 (I)

hat, einer stereochemisch isomeren Form oder einer pharmazeutisch annehmbaren Säureadditionssalzform hievon, worin L für einen Rest der Formel

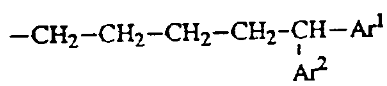 (a),

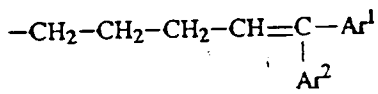 (b),

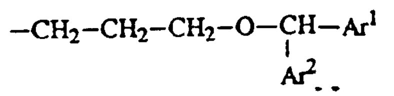 (c),

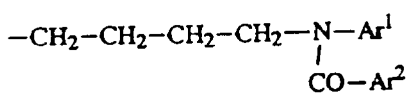 (d),

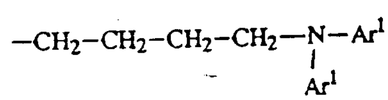 (e),

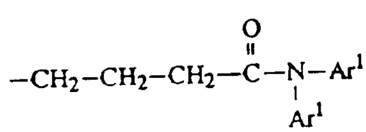 (f),

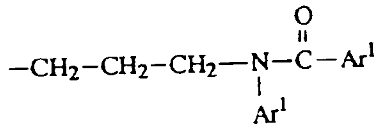 (g)

oder

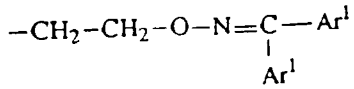 (h)

steht,

Ar[1] für gegebenenfalls durch Halogen oder $C_{1-4}$Alkyloxy substituiertes Phenyl steht;

Ar[2] für gegebenenfalls durch Halogen oder $C_{1-4}$Alkyloxy substituiertes Phenyl oder Pyridinyl steht;

$R^1$ für $C_{1-4}$Alkyl, Aminocarbonyl oder ($C_{1-4}$Alkyl)aminocarbonyl steht;

Ar für einen Rest der Formel

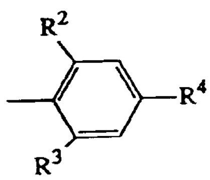 (i), 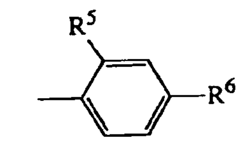 (j), 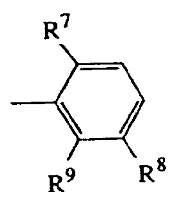 (k),

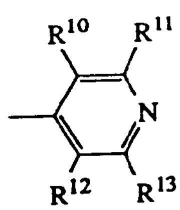 (l) oder 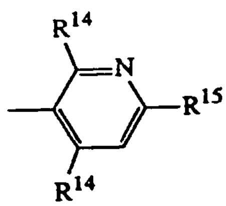 (m)

steht;

| | |
|---|---|
| $R^2$ und $R^3$ | jeweils unabhängig voneinander für Halogen oder $C_{1-4}$Alkyl stehen; |
| $R^4$ | für Wasserstoff, Halogen, Nitro, Amino, Mono- oder Di($C_{1-4}$alkyl)amino, $C_{1-4}$-Alkylcarbonylamino, Aminocarbonylamino, $C_{1-4}$Alkyl, $C_{1-4}$Alkyloxy, $C_{1-4}$Alkyloxycarbonyl, $C_{1-4}$Alkylcarbonyl, Aminocarbonyl, Mono- oder Di($C_{1-4}$Alkyl)aminocarbonyl, Cyano oder Aminomethyl steht; |
| $R^5$ | für $C_1$-4Alkylcarbonyl steht; |
| $R^6$ | für Wasserstoff, Amino, Mono- oder Di($C_{1-4}$alkyl)-amino, $C_{1-4}$Alkylcarbonylamino, Aminocarbonylamino, Aminocarbonyl oder Cyano steht; |
| $R^7$ | für $C_{1-4}$Alkyl steht; |
| $R^8$ | für Halogen oder $C_{1-4}$Alkylcarbonyl steht; |
| $R^9$ | für Wasserstoff oder $C_{1-4}$Alkyl steht; |
| $R^{10}$ | für Halogen oder $C_{1-4}$Alkyl steht; |
| $R^{11}$ | für Wasserstoff, Hydroxy oder $C_{1-4}$Alkyl steht; |
| $R^{12}$ | für Halogen oder $C_{1-4}$Alkyl steht; |
| $R^{13}$ | für Wasserstoff steht; oder |
| $R^{12}$ und $R^{13}$ | zusammengenommen auch einen $C_{3-5}$Alkandiyl-Rest bilden können; |
| | jeder Rest $R^{14}$ für $C_{1-4}$Alkyl steht; und |

$R^{15}$ für $C_{1-4}$Alkyl oder Amino steht.

2. Verwendung einer Verbindung nach Anspruch 1 für die Herstellung eines Medikamentes zum Schützen eines Spenderherzens für die Transplantation während der Lagerung.

3. Verwendung nach Anspruch 1 oder 2, wobei die Verbindung (-)-2-(Aminocarbonyl)-N-(4-amino-2,6-dichlorphenyl)-4[5,5-bis(4-fluorphenyl)pentyl]-1-piperazinacetamidmonohydrat ist.

4. Verfahren zum Schützen eines Spenderherzens für die Transplantation während der Lagerung, welches Verfahren das Herbeiführen des Stillstandes des für die Transplantation vorgesehenen Herzens mit einer Kardioplegie-Lösung und das Lagern dieses Herzens in der Kälte in einer KardioplegieLösung umfaßt, wobei die genannte Kardioplegie-Lösung eine wirksame, das Spenderherz schützende Menge einer Verbindung mit der oben im Anspruch 1 definierten Formel (I) enthält.

5. Verfahren nach Anspruch 4, wobei die Verbindung (-)-2-(Aminocarbonyl)-N-(4-amino-2,6-dichlorphenyl)-4-[5,5-bis(4-fluorphenyl)pentyl]-1-piperazinacetamidmonohydrat ist.

## Revendications

1. Utilisation d'un composé N-aryl-pipérazine-alcanamide pour fabriquer un médicament servant à prévenir ou à limiter les dégâts de ré-irrigation produits lors de la ré-irrigation d'un coeur transplanté, ledit composé ayant pour formule

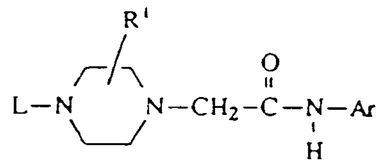 (I),

ou forme stéréo-isomère de celui-ci ou un sel d'addition avec un acide pharmaceutiquement acceptable de celui-ci, formule dans laquelle

L est un radical de formule

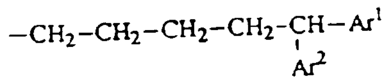 (a),

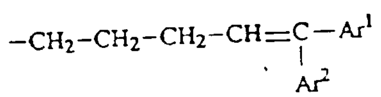 (b),

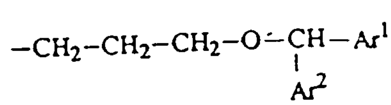 (c),

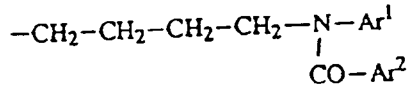 (d),

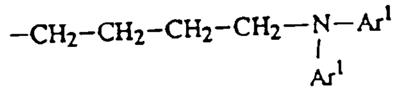

(e),

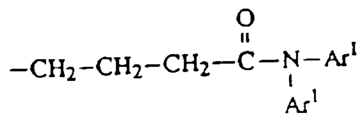

(f),

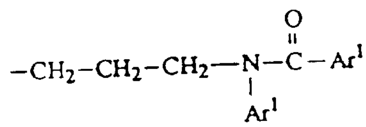

(g)

ou

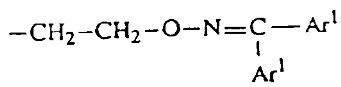

(h),

$Ar^1$ est un groupe phényle, éventuellement substitué par un groupe halogéno ou alkyloxy en $C_1$-$C_4$;

$Ar^2$ est un groupe phényle, éventuellement substitué par un groupe halogéno ou alkyloxy en $C_1$-$C_4$, ou un groupe pyridinyle;

$R^1$ est un groupe alkyle en $C_1$-$C_4$, aminocarbonyle ou (alkyl en $C_1$-$C_4$)aminocarbonyle;

Ar est un radical de formule

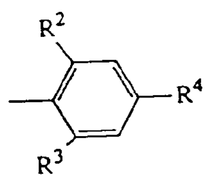

(i),

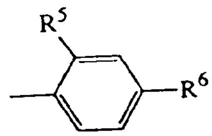

(j),

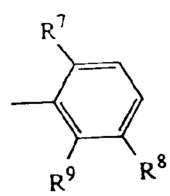

(k),

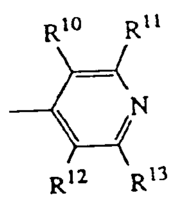

(l) ou

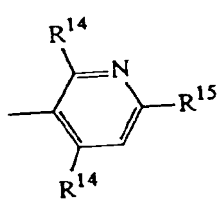

(m)

$R^2$ et $R^3$ sont chacun, indépendamment, un groupe halogéno ou alkyle en $C_1$-$C_4$;

$R^4$ est un atome d'hydrogène, d'halogène, un groupe nitro, amino, mono- ou di(alkyl en $C_1$-$C_4$)amino, alkyl(en $C_1$-$C_4$)carbonylamino, aminocarbonylamino, alkyle en $C_1$-$C_4$, alkyloxy en $C_1$-$C_4$, alkyl(en $C_1$-$C_4$)oxycarbonyle, alkyl(en $C_1$-$C_4$)carbonyle, aminocarbonyle, mono- ou di(alkyl en $C_1$-$C_4$)aminocarbonyle, cyano ou aminométhyle;

$R^5$ est un groupe (alkyl en $C_1$-$C_4$)carbonyle;

$R^6$ est un atome d'hydrogène, un groupe amino, mono- ou di(alkyl en C1-$C_4$)amino, (alkyl en $C_1$-$C_4$)carbonylamino, aminocarbonylamino, aminocarbonyle ou cyano;

$R^7$ est un groupe alkyle en $C_1$-$C_4$;

$R^8$ est un groupe halogéno ou (alkyl en $C_1$-$C_4$)carbonyle;

$R^9$ est un atome d'hydrogène ou un groupe alkyle en $C_1$-$C_4$;

$R^{10}$ est un atome d'halogène ou un groupe alkyle en $C_1$-$C_4$;
$R^{11}$ est un atome d'hydrogène ou un groupe hydroxy ou alkyle en $C_1$-$C_4$;
$R^{12}$ est un atome d'halogène ou un groupe alkyle en $C_1$-$C_4$;
$R^{13}$ est un atome d'hydrogène ou
$R^{12}$ et $R^{13}$, pris conjointement, peuvent aussi former un radical alcane-diyle en $C_3$-$C_5$;
chaque $R^{14}$ est un groupe alkyle en $C_1$-$C_4$; et
$R^{15}$ est un groupe alkyle en $C_1$-$C_4$ ou amino.

**2.** Utilisation d'un composé selon la revendication 1, pour fabriquer un médicament servant à protéger le coeur d'un donneur à transplanter au cours du stockage.

**3.** Utilisation selon la revendication 1 ou 2, dans laquelle le composé est le (-)-2-(aminocarbonyl)-N-(4-amino-2,6-dichlorophényl)-4-[5,5-bis(4-fluorophényl)pentyl]-1-pipérazineacétamide monohydraté.

**4.** Procédé pour protéger le coeur d'un donneur à transplanter au cours du stockage, ledit procédé comprenant l'arrêt du coeur à transplanter avec une solution cardioplegique et le stockage dudit coeur au froid dans une solution cardioplégique, ladite solution cardioplégique comprenant une quantité efficace pour protéger le coeur du donneur d'un composé de formule (I) tel que défini dans la revendication 1.

**5.** Procédé selon la revendication 4, dans lequel le composé est le (-)-2-(aminocarbonyl)-N-(4-amino-2,6-dichlorophényl)-4-[5,5-bis(4-fluorophényl)pentyl]-1-pipérazineacetamide monohydraté.